# EUROPÄISCHE PATENTSCHRIFT

(11) **EP 2 525 746 B1**
(45) Veröffentlichungstag und Bekanntmachung des Hinweises auf die Patenterteilung: **01.05.2013**
(21) Anmeldenummer: 11711012.2
(22) Anmeldetag: 17.01.2011
(51) Int. Cl.: A61F 2/72, A61F 2/78

(54) **LINER MIT INTERGRIERTER ELEKTRODE**
LINER WITH INTEGRATED ELECTRODE
MANCHON À ÉLECTRODE INTÉGRÉE

(30) Priorität: 20.01.2010 DE 102010005462
(43) Veröffentlichungstag der Anmeldung: 28.11.2012
(73) Patentinhaber: Otto Bock HealthCare GmbH, 37115 Duderstadt (DE)
(72) Erfinder: LEINIGER, Andreas, 37327 Leinefelde (DE); VOLKMAR, Jens, 37115 Duderstadt (DE); GÖRTH, Mathias, 37136 Seulingen (DE)
(74) Vertreter: Lins, Edgar
(86) Internationale Anmeldenummer: PCT/DE2011/000055
(87) Internationale Veröffentlichungsnummer: WO 2011/088822

(56) Entgegenhaltungen:
- DE-A1-102005 021 412
- DE-A1-102007 035 409
- US-A- 5 571 208
- US-A1- 2009 216 339

## Beschreibung

Die Erfindung betrifft einen Liner, der zum Überziehen über einen Amputationsstumpf vorgesehen ist, aus einem elektrisch nicht leitenden Material besteht, eine an den Amputationsstumpf angepasste oder sich anpassende Form aufweist, eine an der Haut des Amputationsstumpfes zur Anlage kommende Innenwand aufweist und mit wenigstens einer Elektrode versehen ist, die zur Übertragung elektrischer Signale zwischen der Haut des Amputationsstumpfes und der Außenseite des Liners angeordnet ist.

Liner der hier angesprochenen Art weisen eine gewisse Wandstärke auf und haben die Funktion, eine polsternde und sich an den Amputationsstumpf anpassende oder angepasste Zwischenschicht zwischen dem Amputationsstumpf und der Innenwand eines Prothesenschafts auszubilden. Der Prothesenschaft ist Teil einer Prothese, die den amputierten Teil einer Extremität des Patienten ersetzt.

Die Übertragung elektrischer Signale zwischen dem Amputationsstumpf und der Außenseite des Liners kann aus mehreren Gründen in Betracht kommen. So kann es sinnvoll sein, elektrische Signale von der Haut des Amputations-stumpfes nach außen zu übertragen, um die Funktion der Prothese zu steuern. In diesem Fall können die Elektroden myoelektrische Elektroden sein, die an geeigneten Punkten des Amputationsstumpfes Muskelkontraktionssignale abnehmen, wodurch die Steuerung entsprechender Prothesenglieder erfolgen kann. Eine myoelektrische Steuerung von Prothesen ist insbesondere für Arm- und Handprothesen bekannt, kann aber auch für Bein- und Fußprothesen verwendet werden.

Ferner kann es sinnvoll sein, den Oberflächenwiderstand der Haut des Amputationsstumpfes elektrisch zu bestimmten, indem ein Stromfluss zwischen zwei oder mehreren Elektroden bzw. Elektrodenabschnitten gemessen wird. Auf diese Weise kann beispielsweise bestimmt werden, ob die Haut des Amputationsstumpfes innerhalb des Liners transpiriert, wodurch sich der Sitz des Liners auf dem Amputationsstumpf - und damit der Sitz der Prothese - verschlechtern kann. Ferner ist es möglich, mit Elektroden den Anpressdruck des Amputationsstumpfes an der Innenwand des Liners zu bestimmen, um so beispielsweise auf einen Masseschwund des Amputationsstumpfes während des Tragens der Prothese reagieren zu können.

Umgekehrt kann es sinnvoll sein, von der Außenseite des Liners auf die Haut des Amputationsstumpfes elektrische Signale zu übertragen, beispielsweise um eine Muskelkontraktion des Amputationsstumpfes anzuregen, wenn sich der Prothesenträger länger in einer passiven, beispielsweise sitzenden, Stellung befindet.

Durch US 5,443,525 ist ein Liner bekannt, der zur Aufnahme myoelektrischer Elektroden vorgesehen ist. Hierzu wird in ein Fenster des Prothesenschafts ein nicht metallisches, flexibles und weiches flächiges Polster eingeklebt, in dem sich eine große Anzahl von diskreten leitenden Elektroden befindet. Der Liner besteht vorzugsweise aus Silikon, einem nicht leitenden flexiblen Kunststoff. Die Elektroden können aus einer Mischung aus Silikon und Kohlenstoff oder aus Silikon und Silber gebildet sein, wobei die Elektroden jeweils von nicht leitendem Silikon umgeben sind. Die Elektrodenanordnung ist über das Polster somit auf die Innenseite des Liners geklebt und ist durch das Fenster des Liners zugänglich, sodass die von den Elektroden abgenommenen myoelektrischen Signale durch das Fenster nach außen zur Auswertung bzw. Steuerung geleitet werden können. Diese Anordnung ist aufwendig herzustellen und weist einen begrenzten Tragekomfort auf. Darüber hinaus bedarf das Fenster des Liners eines besonderen Abdichtungsaufwands, wenn der Liner - wie häufig üblich - luftdicht ausgebildet sein muss, um mit Hilfe eines im Innenraum des Liners ausgebildeten Unterdrucks den Liner am Amputationsstumpf zu halten. Der Unterdruck muss dabei vom Liner gegen das Gewicht der bewegten Prothese aufrechterhalten werden.

Aus der US 2009/0216339 ist ein Liner bekannt, in dem Kontakte aus leitendem Material eingesetzt sind.

Der vorliegenden Erfindung liegt daher die Aufgabe zugrunde, einen Liner der eingangs erwähnten Art so auszubilden, dass ein hoher Tragekomfort und eine sichere Kontaktierung durch die Elektroden gewährleistet ist.

Zur Lösung dieser Aufgabe ist erfindungsgemäß ein Liner der eingangs erwähnten Art dadurch gekennzeichnet, dass im Bereich der Elektrode wenigstens ein leitender Abschnitt in das Material des Liners integriert ist, der mit dem nicht leitenden Material des Liners eine einheitliche, stetig ausgerichtete Innenwand bildet.

Der erfindungsgemäße Liner weist somit wenigstens einen leitenden Abschnitt für eine Elektrode auf, der Teil des Materials des Liners ist, sodass eine einheitliche, stetige Innenwand vorhanden ist, die sich von der Innenwand eines Liners ohne Elektroden nicht unterscheidet. Bevorzugt ist dabei das Material des Liners ein Polymerisat, beispielsweise Silikon. Der leitende Abschnitt ist dabei vorzugsweise vor dem Auspolymerisieren in das Material eingesetzt worden, sodass der leitende Abschnitt beim Auspolymerisieren des Materials des Liners mit diesem zu einem einheitlichen Teil verbunden wird, wobei sich die stetige, glatte Innenwand ausbildet.

Dabei ist es vorteilhaft, wenn der leitende Abschnitt aus dem Material des Liners besteht, das mit Zusätzen elektrisch leitfähig gemacht worden ist, wie dies grundsätzlich beispielsweise aus der US 5,443,525 bekannt ist. Dabei ist es ferner möglich, wenn auch nicht notwendig, dass der leitende Abschnitt gemeinsam mit dem Material des Liners auspolymerisiert ist.

Der leitende Abschnitt des Liners kann selbst als Elektrode ausgebildet sein, also beispielsweise mit einer Leitung kontaktiert sein, mit oder ein abgenommenes elektrisches Signal zu einer Steuerung geleitet wird oder ein elektrisches Signal als Anregungssignal für den Amputationsstumpf auf dessen Haut übertragen wird.

In einer bevorzugten Ausführungsform dient der leitende Abschnitt des Liners jedoch als Kontaktvermittler für eine Elektrode, die außenseitig an dem leitenden Abschnitt angebracht ist. In diesem Fall bildet der leitende Abschnitt vorzugsweise eine dünne Wandung einer im Material des Liners ausgebildeten Ausnehmung, die zur Aufnahme der Elektrode dient. Dadurch ist eine genaue und sichere Positionierung der Elektrode an einer vorbestimmten Stelle des Liners möglich.

Die Ausnehmung kann einen durch eine Materialverdickung gebildeten Rand aufweisen, wodurch einerseits die Einbringung der Elektrode stabilisiert wird und andererseits die Elektrode ggf. über ihre gesamte Dicke geschützt ist. Dabei kann der Rand mit Hinterschneidungen zur Aufnahme entsprechend vorstehender Ränder der Elektrode versehen sein, sodass der Rand zugleich der sicheren Befestigung der Elektrode in der Aufnahme dient. Die Hinterschneidungen sind dabei vorzugsweise so angebracht, dass die Elektrode mit einer gewissen Vorspannung gegen den leitenden Abschnitt gedrückt wird, um eine sicher Kontaktierung zu gewährleisten.

Die Elektrode ist vorzugsweise mit einer das elektrische Signal abführenden Leitung versehen, die zumindest teilweise im Material des Liners geführt ist. Hierzu kann der Liner nach außen offene Einschnitte aufweisen, in die die Leitung eingelegt werden kann. Alternativ ist es möglich, wenigstens eine Leitung von der Ausnehmung zu einer geeigneten Stelle des Liners, vorzugsweise an einem geschlossenen distalen Ende des Liners, in den Liner bei dessen Herstellung bereits zu integrieren. In diesem Fall ist eine geeignete Kontaktvorrichtung für die Elektrode in der Aufnahme vorzusehen. Diese kann im einfachsten Fall aus einem vorstehenden unisolierten Teil der Leitung gebildet sein, der mit der in die Aufnahme eingesetzten Elektrode verbindbar ist, beispielsweise mit einem Steckanschluss, wie er für den Anschluss von Lampenkabeln üblich ist, durch Schraubkontaktklemmen oder auch durch Löten.

Der erfindungsgemäße Liner lässt sich in einem nur leicht modifizierten herkömmlichen Herstellungsverfahren produzieren, sodass die Ausbildung des Liners mit den Elektroden oder zur Aufnahme von Elektroden ohne großen Zusatzaufwand und erhebliche Zusatzkosten möglich ist.

Die Erfindung soll im Folgenden anhand von in der Zeichnung dargestellten Ausführungsbeispielen näher erläutert werden. Es zeigen:
- Figur 1: eine Draufsicht auf einen Abschnitt eines Liners mit einer Aufnahme für eine Elektrode;
- Figur 2: einen Querschnitt durch den Liner gemäß Figur 1;
- Figur 3: ein vergrößertes Detail A der Figur 2;
- Figur 4: einen Längsschnitt durch den Liner gemäß Figur 1;
- Figur 5: eine vergrößerte Darstellung des Details B aus Figur 4;
- Figur 6: eine perspektivische Ansicht eines am distalen Ende geschlossen ausgebildeten Liners mit einer Mehrzahl von Elektroden oder Aufnahme für Elektroden sowie in den Liner eingebrachten Verbindungsleitungen zu einem Anschlussstecker, der am distalen Ende des Liners angebracht ist.

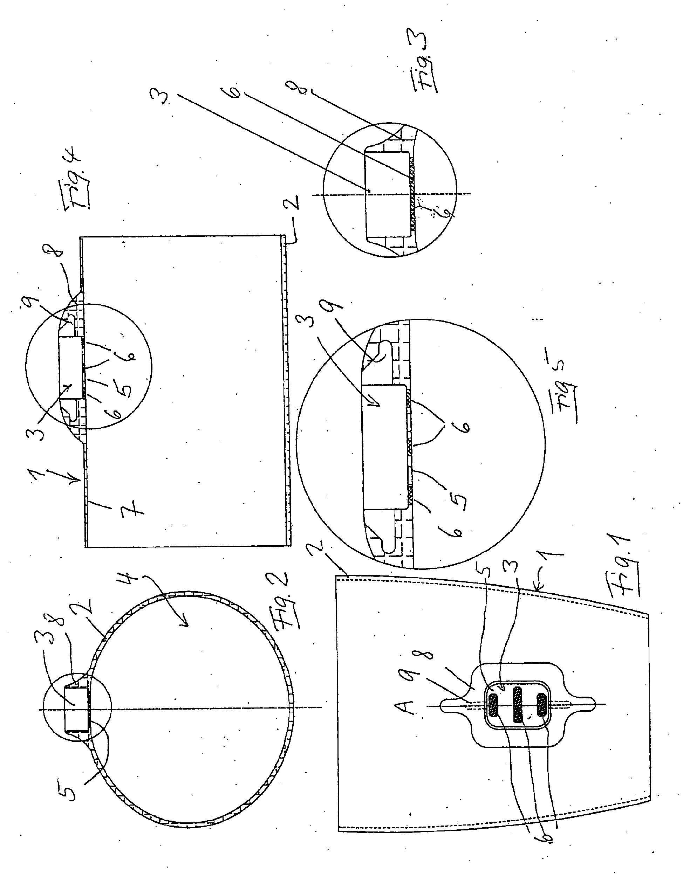

Der in den Figuren 1 bis 5 dargestellte Abschnitt eines Liners lässt erkennen, dass dieser mit einer nahtlos geschlossen Wandung 2 ausgebildet ist und sich in üblicher Weise trichterförmig zu einem distalen Ende hin verjüngt. In das Material der Wandung 2 ist eine Aufnahme 3 in Form einer nach radial außen offenen Kammer ausgebildet die zu einem Innenraum 4 des Liners hin mit einem dünnen Wandstück 5 als Boden der Ausnehmung 3 abgeschlossen ist. In dem Wandstück 5 befinden sich drei leitende Abschnitte 6, die mit Abstand zueinander und durch das nicht leitende Material des Liners 1 voneinander getrennt sind. Die leitenden Abschnitte sind einstückig mit dem Material des Wandstücks 5 ausgebildet, sodass eine glatte und stetige Innenwand 7 auch im Bereich der Aufnahme 3 vorhanden ist, wie dies insbesondere aus den Figuren 3 und 5 hervorgeht.

Die Figuren 2 bis 5 verdeutlichen, dass die Aufnahme 3 durch einen Rand 8 begrenzt ist, der durch eine Materialverdickung der Wandung 2 des Liners 1 gebildet ist.

Figur 1 verdeutlicht, dass sich der Rand 8 als im Wesentlichen rechteckiger Rahmen in Axialrichtung A länglich erstreckt und dass die drei leitenden Abschnitte 6 in Axialrichtung A hintereinander angeordnet sind. Der in den Figuren 2 und 3 dargestellte Querschnitt erstreckt sich durch den mittleren leitenden Abschnitt hindurch, während die Längsschnitte der Figuren 4 und 5 in der Ebene der Axialrichtung A in Figur 1 dargestellt sind.

Die Figuren 4 und 5 verdeutlichen ferner, dass in dem Rand 8 in Axialrichtung A zeigende Hinterschneidungen 9 eingebracht sind, in die entsprechend geformte vorstehende Ränder einer Elektrode aufgrund der Flexibilität des Materials der Wandung 2 bzw. der Ränder 8 einsetzbar sind. Auf diese Weise dient der Rand 8 zugleich der sicheren und geschützten Befestigung einer Elektrode in der Aufnahme 3.

Die in den Figuren 1 bis 5 dargestellten Ausführungsform sieht somit leitende Abschnitte 6 vor, die als leitendes Übertragungsmittel zwischen der Haut des in den Liner 1 eingebrachten Amputationsstumpfes und einer in die Aufnahme 3 eingebrachten Elektrode dient. Diese Ausführungsform ist gegenüber der un-mittelbaren Ausbildung der leitenden Abschnitte 6 selbst als Elektrode bevorzugt.

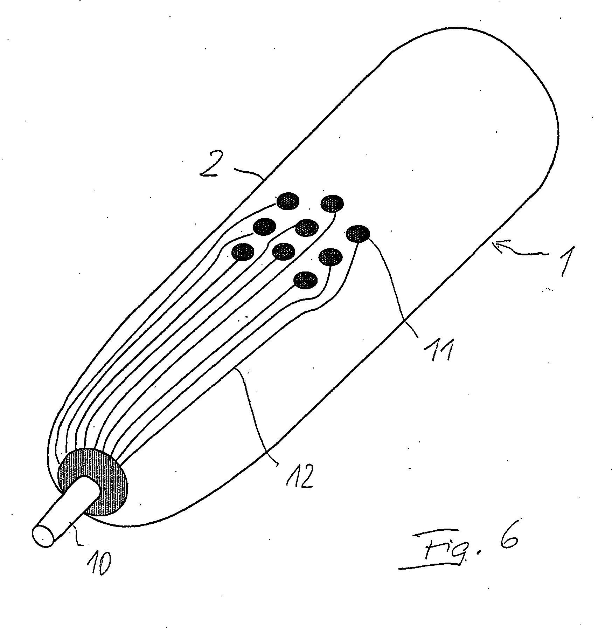

Figur 6 zeigt in einer perspektivischen Darstellung den vollständigen trichterförmigen Liner 1, der zu seinem distalen Ende hin geschlossen ausgebildet ist und dort einen schematisch angedeuteten vielpoligen Anschlussstecker 10 aufweist. In die Wandung 2 des Liners sind eine Vielzahl von Elektroden 11 eingebracht, die gemäß der Darstellung der Figuren 1 bis 5 in entsprechende Aufnahmen 3 eingesetzt sein können. Von den Elektroden 11 erstrecken sich in der Wandung 2 des Liners Verbindungsleitungen 12 in axialer Richtung zum Stecker 10 am distalen Ende des Liners 1. Die Verbindungsleitungen 12 sind vorzugsweise in die Wandung 2 des Liners 1 bei dessen Herstellung eingebracht, also mit dem Liner 1 vorproduziert. Dies ist insbesondere von Vorteil, wenn die Elektroden 11 nicht unmittelbar mit der zugehörigen Verbindungsleitung 12 verbunden ist, sondern durch Einsetzen in eine Aufnahme 3 mit der Verbindungsleitung 12 kontaktierbar ist. Der elektrische Kontakt der Elektrode 11 mit der Haut des Patienten erfolgt in jedem Fall über die leitenden Abschnitte 6 in dem dünnen Wandstück 5 der Aufnahme 3.

In die Aufnahme 3 muss nicht eine einheitliche Elektrode 11 eingesetzt werden. Vielmehr kann die Elektrode 11 auch aus unterschiedlichen Elektrodenabschnitten bestehen, die mit den mehreren leitenden Abschnitten 6 fluchten. Dadurch kann beispielsweise der Stromfluss zwischen zwei leitenden Abschnitten 6 gemessen werden, um die Leitfähigkeit der an der Innenwand 7 des Liners anliegenden Haut des Amputationsstumpfes zu bestimmen.

In ähnlicher Weise können zahlreiche Messungen an der Haut des Amputationsstumpfes ausgeführt werden. Insbesondere kann der erfindungsgemäße Liner 1 auch zur Abnahme von myoelektrischen Signalen von dem Amputationsstumpf verwendet werden.

Eine weitere Anwendungsmöglichkeit ergibt sich durch die Übertragung von elektrischen Stimulationssignalen von der Außenseite des Liners 1 auf die Haut des in den Liner 1 eingesetzten Amputationsstumpfes.

Der erfindungsgemäße Liner weist eine glatte und stetige Innenwandung 7 auf und unterscheidet sich von seinen Trageigenschaften nicht von herkömmlichen Linern, die ohne Elektroden ausgebildet sind.

## Patentansprüche

1. Liner, der zum Überziehen über einen Amputationsstumpf vorgesehen ist, aus einem elektrisch nicht leitenden Material besteht, eine an den Amputationsstumpf angepasste oder sich anpassende Form aufweist, eine an der Haut des Amputationsstumpfes zur Anlage kommende Innenwand (7) aufweist und mit wenigstens einer Elektrode (11) versehen ist, die zur Übertragung elektrischer Signale zwischen der Haut des Amputationsstumpfes und der Außenseite des Liners (1) angeordnet ist, **dadurch gekennzeichnet, dass** im Bereich der Elektroden (11) wenigstens ein leitender Abschnitt (6) in das Material des Liners (1) integriert ist, der mit dem nicht leitenden Materials des Liners (1) eine einheitliche und stetig ausgerichtete Innernwand bildet.

2. Liner nach Anspruch 1, **dadurch gekennzeichnet, dass** das Material des Liners (1) ein Polymerisat ist und dass der leitende Abschnitt (6) vor dem Auspolymerisieren in das Material eingesetzt ist.

3. Liner nach Anspruch 1 oder 2, **dadurch gekennzeichnet, dass** der leitende Abschnitt (6) aus dem Material des Liners (1) besteht, das mit Zusätzen elektrisch leitfähig gemacht worden ist.

4. Liner nach Anspruch 3, **dadurch gekennzeichnet, dass** der leitende Abschnitt (6) gemeinsam mit dem Material des Liners (1) auspolymerisiert ist.

5. Liner nach einem der Ansprüche 1 bis 4, **dadurch gekennzeichnet, dass** der leitende Abschnitt als Elektrode ausgebildet ist.

6. Liner nach einem der Ansprüche 1 bis 4, **dadurch gekennzeichnet, dass** außenseitig an den leitenden Abschnitt (6) eine Elektrode (11) angebracht ist.

7. Liner nach Anspruch 6, **dadurch gekennzeichnet, dass** der leitende Abschnitt (6) eine dünne Wandung (5) einer im Material des Liners (1) ausgebildeten Ausnehmung (3) zur Aufnahme der Elektrode (11) bildet.

8. Liner nach Anspruch 7, **dadurch gekennzeichnet, dass** die Ausnehmung (3) einen durch eine Materialverdickung gebildeten Rand (8) auf weist.

9. Liner nach Anspruch 8, **dadurch gekennzeichnet, dass** der Rand (8) mit Hinterschneidungen (9) zur Aufnahme entsprechend vorstehender Ränder der Elektrode (11) versehen ist.

10. Liner nach einem der Ansprüche 1 bis 9, **dadurch gekennzeichnet, dass** die Elektrode (11) mit einer das elektrische Signal führenden Leitung (12) versehen ist, die zumindest teilweise im Material des Liners (1) geführt ist.

## Claims

1. A liner which is intended to be pulled over an amputation stump, is made of an electrically non-conducting material, has a shape that is adapted or that adapts to the amputation stump, has an inner wall (7) coming to lie against the skin of the amputation stump, and is provided with at least one electrode (11) which is arranged to transmit electrical signals between the skin of the amputation stump and the outside of the liner (1), **characterized in that** at least one conducting section (6) is integrated into the material of the liner (1) in the area of the electrodes (11) and, together with the non-conducting material of the liner (1), forms a uniform and continuously oriented inner wall.

2. The liner as claimed in claim 1, **characterized in that** the material of the liner (1) is a polymer, and **in that** the conducting section (6) is inserted into the material before polymerization to completion.

3. The liner as claimed in claim 1 or 2, **characterized in that** the conducting section (6) is made from the material of the liner (1) , which material has been made electrically conductive by additives.

4. The liner as claimed in claim 3, **characterized in that** the conducting section (6) is polymerized to completion together with the material of the liner (1).

5. The liner as claimed in one of claims 1 through 4, **characterized in that** the conducting section is designed as an electrode.

6. The liner as claimed in one of claims 1 through 4, **characterized in that** an electrode (11) is applied to the outside of the conducting section (6).

7. The liner as claimed in claim 6, **characterized in that** the conducting section (6) forms a thin wall (5) of a recess (3) that is formed in the material of the liner (1) and that receives the electrode (11).

8. The liner as claimed in claim 7, **characterized in that** the recess (3) has an edge (8) formed by a material thickening.

9. The liner as claimed in claim 8, **characterized in that** the edge (8) is provided with undercuts (9) for receiving correspondingly protruding edges of the electrode (11).

10. The liner as claimed in one of claims 1 through 9, **characterized in that** the electrode (11) is provided with a lead (12) that carries the electrical signal and that is routed at least in part in the material of the liner (1).

## Revendications

1. Manchon, qui est prévue pour être tirée par-dessus un moignon d'amputation, qui est en un matériau non conducteur de l'électricité, qui présente une forme adaptée au moignon d'amputation ou une forme qui s'y adapte, qui comporte une paroi intérieure (7) venant en contact avec la peau du moignon d'amputation, et qui est pourvue d'au moins une électrode (11), agencée pour la transmission de signaux électriques entre la peau du moignon d'amputation et la face extérieure de la manchon (1),
**caractérisée en ce que** dans la région des électrodes (11) au moins un tronçon conducteur (6) est intégré dans le matériau de la manchon (1), tronçon qui forme avec le matériau non conducteur de la manchon (1) une paroi intérieure unitaire et constamment orientée.

2. Manchon selon la revendication 1, **caractérisée en ce que** le matériau de la manchon (1) est un polymérisat, et **en ce que** le tronçon conducteur (6) est mis en place dans le matériau avant la polymérisation.

3. Manchon selon la revendication 1 ou 2, **caractérisée en ce que** le tronçon conducteur (6) est fait du matériau de la manchon (1), qui a été rendu électriquement conducteur avec des additifs.

4. Manchon selon la revendication 3, **caractérisée en ce que** le tronçon conducteur (6) est polymérisé conjointement avec le matériau de la manchon (1).

5. Manchon selon l'une des revendications 1 à 4, **caractérisée en ce que** le tronçon conducteur est réalisé comme une électrode.

6. Manchon selon l'une des revendications 1 à 4, **caractérisée en ce qu'**une électrode (11) est appliquée du côté extérieur sur le tronçon conducteur (6).

7. Manchon selon la revendication 6, **caractérisée en ce que** le tronçon conducteur (6) forme une paroi mince (5) d'un évidement (3) ménagé dans le matériau de la manchon (1) pour recevoir l'électrode (11).

8. Manchon selon la revendication 7, **caractérisée en ce que** l'évidement (3) comporte une bordure (8) formée par un épaississement du matériau.

9. Manchon selon la revendication 8, **caractérisée en ce que** la bordure (8) est pourvue de contre-dépouilles (9) pour recevoir des bordures saillantes correspondantes de l'électrode (11).

10. Manchon selon l'une des revendications 1 à 9, **caractérisée en ce que** l'électrode (11) est pourvue d'une ligne (12) menant le signal électrique, qui est guidée au moins partiellement dans le matériau de la manchon (1).
